# EUROPEAN PATENT SPECIFICATION

(11) **EP 2 766 083 B1**
(45) Date of publication and mention of the grant of the patent: **29.05.2019**
(21) Application number: 12840613.9
(22) Date of filing: 12.10.2012
(51) Int. Cl.: A61M 25/092, A61M 25/088, A61B 10/02

(54) **CATHETER SYSTEMS**
KATHETERSYSTEME
SYSTÈMES CATHÉTER

(30) Priority: 14.10.2011 US 201113274198; 14.10.2011 US 201113274237; 14.10.2011 US 201113274229; 14.10.2011 US 201113274208
(43) Date of publication of application: 20.08.2014
(73) Proprietor: Intuitive Surgical Operations, Inc., Sunnyvale, CA 94086 (US)
(72) Inventor: AU, Samuel Kwok Wai, Mountain View, California 94043 (US); DONHOWE, Caitlin Q., Sunnyvale, California 94089 (US); DUINDAM, Vincent, Sunnyvale, California 94086 (US); FENECH, Carolyn, San Jose, California 95124 (US); MOHR, Catherine J., Mountain View, California 94040 (US); PRISCO, Giuseppe Maria, I-56025 Pontidera (PI) (IT)
(74) Representative: MacDougall, Alan John Shaw
(86) International application number: PCT/US2012/059889
(87) International publication number: WO 2013/056006

(56) References cited:
- WO-A1-02/07809
- WO-A2-2004/016155
- WO-A2-2007/109418
- US-A1- 2005 197 557
- US-A1- 2007 049 908
- US-A1- 2007 265 503
- US-A1- 2008 097 155
- US-A1- 2010 076 303
- US-A1- 2010 222 647

## Description

### BACKGROUND

Medical devices that navigate body lumens need to be physically small enough to fit within the lumens. Lung catheters, for example, which may be used to perform minimally invasive lung biopsies or other medical procedures, may need to follow airways that decrease in size as the catheter navigates branching passages. To reach a target location in a lung, a catheter may follow passages having diameters as small as 3 mm or less. Manufacturing a catheter that includes the mechanical and sensor structures suitable for remote or robotic operation and that has a diameter that is sufficiently small to navigate such small lumens can be challenging. In particular, one desirable configuration for a remotely operated catheter would provide a tool mounted on a steerable segment; tendons or pull wires that extend down the length of the catheter to an external drive system that pulls on the tendons to actuate the tool or steerable segment; lumens for suction and/or irrigation; a vision system for viewing of the target location; and sensors to identify the location of the instrument relative to the anatomy of a patient. Accommodating all of the desired features and elements of a lung catheter or other device that is robotically controlled and has a diameter about 3 mm or less can be difficult.

US 2007/265503 discloses a medical instrument system comprising an elongate instrument body; an optical fiber coupled in a constrained manner to the elongate instrument body, the optical fiber including one or more Bragg gratings; a detector operably coupled to a proximal end of the optical fiber and configured to detect respective light signals reflected by the one or more Bragg gratings; and a controller operatively coupled to the detector, wherein the controller is configured to determine a geometric configuration of at least a portion of the elongate instrument body based on a spectral analysis of the detected reflected portions of the light signals.

US 2005/197557 discloses a system for guiding a catheter through a lumen system of a body of a patient, to a predetermined location within the lumen system, the system including a medical positioning system, a moving mechanism coupled with the catheter, and a controller coupled with the medical positioning system and with the moving mechanism, the medical positioning system including at least one position detector, the position detector being firmly attached to a distal portion of the catheter, the medical positioning system determining the position of the position detector, the controller controlling the operation of the moving mechanism to move the catheter to the predetermined location, according to the position and according to a topological representation of at least a portion of the lumen system.

US2012/123441 discloses control systems and methods for a medical instrument which use measurements to determine and control the tensions that actuators apply through instrument transmission systems. One embodiment determines joint torques and tensions from differences between desired and measured joint positions. Another embodiment determines joint torques and tensions from differences between desired and measured positions of a tip of the instrument. Determination of tensions from joint torques can be performed using sequential evaluation of joints in an order from a distal end of the instrument toward a proximal end of the instrument.

### SUMMARY

The invention is set out in the independent claim 1, and preferred embodiments are indicated in the dependent claims that follow.

In accordance with an example, a catheter control system has a control mode (sometimes referred to herein as a holding mode) that actively maintains the catheter in a working configuration desired for a medical procedure. This holding mode facilitates use of the catheter system with interchangeable probes. In particular, a vision probe can be deployed in the catheter while the catheter navigates to a work site, to view the work site, or to assist in identifying the desired working configuration for the catheter during performance of a medical task. The catheter control system can then switch into the holding mode, and the vision system can be removed. A medical probe can then be deployed through the catheter in place of the removed vision probe, and the control system maintains the working configuration and allows performance of a medical task without the vision probe, while the desired working configuration is fixed and held. In one implementation, the control system actively keeps the catheter in the desired working configuration while the medical probe is inserted through the catheter, reaches the work site, and performs a medical function. In an alternative implementation, the control system returns the catheter to the recorded working configuration before the medical function is performed. Since the catheter only needs to accommodate the vision or medical probe and not both, the diameter of the catheter may be smaller than might otherwise be possible for a system that provides similar functionality.

In accordance with another example, a feedback control method and system for a robotically controlled flexible device implements multiple different modes of closed-loop device actuation or stiffening for different applications and usage scenarios. The different stiffening modes can cause the device to respond in a desired way in case of externally applied forces, for example, tissue reaction forces, as the device navigates through a clinical space or as the device interacts with tissue as part of a medical procedure.

In one embodiment, a flexible device such as a catheter uses real-time feedback from a sensor system in generation of signals for actuators attached to tendons that are used to articulate a distal portion of the device. For example, a catheter may include a flexible section at its distal tip that can bend in two directions (pitch and yaw). A fiber-optic shape sensor can measure the bending of the flexible section and return measurement data indicating the position and orientation of the distal tip relative to a base of the shape sensed. The position of the base may be determined, for example, using electromagnetic sensors that provide measurements of a position and orientation of the base relative to an external reference that may be attached to a patient. Multiple actuation tendons (e.g., pull wires or cables) attach to the distal tip and run along the length of the catheter to the actuators. Control logic that controls the actuators to pull the tendons and hence move the distal tip in any pitch/yaw direction can operate in different modes for different purposes. In particular, for a holding mode, the control logic can use the sensor data and fixed information on a target shape of the catheter to compute control signals for the actuators.

The control logic for a robotic catheter or other flexible system may have multiple modes of operation including one or more of the following: 1.) A position stiffness mode in which the control system controls actuators to minimize a difference between desired and measured positions of the distal tip of the catheter or probe; 2.) An orientation stiffness mode in which the control system controls the actuators to minimize a difference between desired and measured pointing directions of the distal tip; 3.) A target position stiffness mode in which the control system uses a combination of the measured tip position and pointing direction to control the distal tip to always point towards a specified target point; and 4.) A target axial motion stiffness mode in which the control system uses a combination of the measured tip position and pointing direction, together with sensor measurements from other parts of the device, and controls actuators to ensure that the distal tip is positioned on a specific line in space and has a pointing direction also along that line. The selection of which of the available modes the control system uses can be made through user selection, according to the type of probe being used (e.g. forceps, camera, laser, brush, or needle), or according to the action the catheter is performing (e.g., navigating or performing a biopsy). Any of these modes can be used to hold the catheter for a medical procedure by fixing the desired location, direction, target point, or line.

In accordance with an example, a catheter system has a removable vision probe that can be replaced with a biopsy probe or other medical probe. The vision probe may be used for navigation to a work site and then removed and replaced with the biopsy probe that takes a tissue sample. The catheter system can thus provide a high level of vision and medical functionality in a small diameter. Control logic for robotic actuation of the catheter adjust for issues that may occur if the catheter moves while the tools or probes are swapped or if the patient moves. In particular, when the vision probe is removed, feedback control and a distal sensor (such as a fiber-optic shape sensor) may be used to maintain or return to the desired location or working configuration for use of the biopsy probe. The feedback information from the sensor thus can be used to control the actuator tendons such that the working configuration of a distal tip of the catheter is maintained even under disturbances due to tool removal or anatomy motion. In this way, accuracy of the catheter system is maintained and biopsy yield may be improved. Instead of maintaining a constant working configuration, parameters of the desired working configuration can be recorded, and the catheter can be straightened or relaxed for easier removal and insertion of probes and subsequently returned to the desired working configuration after a new probe has been inserted or upon user indication.

One specific example of the invention is a medical system including a catheter and control logic. The catheter includes a main lumen and a mechanical system. The main lumen accommodates either a vision probe or a medical probe and is too narrow to simultaneously guide both a vision probe and a medical probe to the distal tip. The control logic operates the mechanical system to control a pose of a distal tip of the catheter. In particular, the control logic can identify a desired working configuration of the distal tip while the vision probe is deployed and can hold or return the catheter to the desired working configuration when the vision probe has been removed from the catheter and the medical probe is deployed in the main lumen.

Another specific example of the invention is a medical system including a removable vision probe and a catheter. The catheter has a lumen sized to guide either the vision system or a medical tool to a distal tip of the catheter. Further, the catheter has a width less than about 3 mm and is too narrow to simultaneously guide both the vision probe and the medical probe to the distal tip.

Yet another example is a process that includes: using a vision probe deployed in a catheter when identifying a working configuration of a distal tip of the catheter; removing the vision probe from the catheter; deploying a medical probe in place of the vision probe that was removed; and actuating the catheter to maintain or return to the working configuration of the distal tip when a medical probe is deployed in the catheter in place of the vision probe.

In accordance with an example, functionality for a catheter system is divided between a catheter and a removable vision probe. Particular arrangements can maximize the usefulness of the small overall system cross-section that can fit, for example, in a small diameter lung lumen. In particular, a removable vision probe can provide imaging, illumination, irrigation, and suction utility that may be employed before a biopsy or treatment probe is deployed. The catheter can include actuation and sensing structures that are useful with or necessary for both a vision probe and a medical probe. Accordingly, the catheter can maximize available space for interchangeable probes and still provide all necessary functions for use of a probe such as a medical probe when the vision probe is removed.

In one specific example, a vision probe for a medical system includes a tube containing multiple channels such as a main channel, one or more oblong channels for irrigation and suction, and one or more auxiliary channels. An imaging system extends through the main channel to a distal end of the tube, and one or more illumination fibers extend through respective auxiliary channels in the tube. The tube can optionally be implemented as an extrusion that includes the multiple channels, and in one specific example, the imaging system includes a camera mounted at the distal end of the vision probe.

In accordance with a further example, the medical system can further include a catheter having a lumen in which the vision probe can be deployed and removed during a medical procedure. The catheter may include steering and sensor systems that can be used with the vision probe or probes that replace the vision probe in the catheter.

In accordance with an example, a robotic catheter system using distal feedback can provide a small diameter for a distal tip of the catheter and accurate measurements of the pose of the distal tip through use of a sensor system including both electromagnetic and fiber sensors. In accordance with an example, a sensor system for a catheter has a thicker proximal section containing one or more electromagnetic (EM) sensors and a thinner distal section containing a fiber shape sensor. The EM sensor can provide an accurate measurement of a base point of the distal section relative to the anatomy of a patient, while the fiber sensor measures the shape of the distal section extending from the base point. Accordingly, the distal section of the catheter can be as small as a system using only a fiber shape sensor, but the catheter system does not suffer from the inaccuracy that is common to long fiber shape sensors.

One specific example is a medical system including a catheter, a first sensor system, and a second sensor system. The catheter has a first section and a second section with the second section being adjacent to the first section. The first sensor system is in the first section and configured for measurement of a pose of the first section. The second sensor system is in the second section and configured for measurement of a pose of the second section relative to the first section.

Another example is a method for sensing a pose of a distal tip of an elongated flexible structure such as a catheter in a medical instrument. The method includes applying a time-varying magnetic field to a space containing at least a portion of the flexible structure. An electric signal induced in a coil positioned at a location along the flexible structure of the medical instrument can then be analyzed as part of the pose measure. The location of the coil is separated from a proximal end and the distal tip of the flexible structure. In addition to analysis of the electrical signal from the coil, a shape of a portion of the flexible structure that extends from the location of the coil toward the distal end of the flexible section is measured.

One specific example is a medical system including: a catheter containing a mechanical system that is remotely operable to control a distal tip of the catheter; a sensor configured to at least partly measure a pose of the distal tip; and a control system coupled to the mechanical system. The control system has a plurality of modes of operation including a holding mode in which the control system operates the mechanical system to actively maintain a working configuration of the distal tip based on feedback from the sensor.

The plurality of operating modes of the control system may further include a mode in which the control system operates the mechanical system to steer the catheter in response to user input.

The control system and the sensor may form a closed-loop system for movement and sensing of the working configuration of the distal tip.

The working configuration may define a position of the distal tip relative to a work site or an orientation of the distal tip relative to a work site.

Maintaining the working configuration may include maintaining a combination of a position and an orientation of the distal tip such that the distal tip remains pointed at a target in a work site. With this mode, the medical system may further include a removable probe deployed in the catheter, and the probe may include a laser configured so that the working configuration points the laser at the target.

Maintaining the working configuration may include keeping the distal tip on a target line. With this mode, maintaining the working configuration may further maintain an axis of the distal tip along the target line.

The holding mode of the control system may include multiple sub-modes in which the control system maintains different aspects of the pose of the distal tip, and the control system may include a selection module configured to place the control system in one of the sub-modes that corresponds to a type of probe deployed in the catheter. The sub-modes may include an orientation sub-mode in which the control system maintains an orientation of the distal tip relative to a work site, and the selection module places the control system in the orientation sub-mode when a vision probe is deployed in the catheter. The sub-modes may include a target sub-mode in which the control system maintains a combination of a position and an orientation of the distal tip such that the distal tip remains pointed at a target in a work site, and the selection module can place the control system in the target sub-mode when a probe including a laser is deployed in the catheter. The sub-modes may include a target axial sub-mode in which the control system maintains a combination of a position and an orientation of the distal tip such that the distal tip remains on a target line and pointed along the target line, and the selection module can place the control system in the target axial sub-mode when a probe including a needle is deployed in the catheter.

Another specific example is a control system for a catheter containing a mechanical system that is remotely operable to control a distal tip of the catheter, and a sensor configured to at least partly measure a pose of the distal tip coupled to the mechanical system. The control system includes: multiple modules that control operation the mechanical system based on feedback from the sensor; and memory storing an indicator identifying a desired working for the distal tip. Each of the modules operates the mechanical system to hold the distal tip in the desired working configuration identified by the indicator.

The modules may include: a position mode module that implements a process including moving the distal tip from a measured position to a desired position; an orientation mode module that implements a process including moving the distal tip from a measured orientation to a desired orientation; a target mode module that maintains a combination of a position and an orientation of the distal tip such that the distal tip remains pointed at a target; or an target axial mode module that maintains a combination of a position and an orientation of the distal tip such that the distal tip remains on a target line and pointed along the target line.

Another specific example is a method including: steering a catheter to a working site using a mechanical system that is remotely operable to control a distal tip of the catheter; identifying a working configuration of the distal tip, wherein the working configuration poses the distal tip for performance of a medical task at the work site; selecting one of a plurality of modes for controlling the catheter using feedback from a sensor that measures a pose of the distal tip; and maintaining the working configuration of the distal tip through operation of the catheter in the selected mode.

The modes may include: a position mode that maintains a position of the distal tip relative to the work site; an orientation mode that maintains an orientation of the distal tip relative to the work site; a target mode that maintains a combination of a position and an orientation of the distal tip such that the distal tip remains pointed at a target in the work site; or an target axial mode that maintains a combination of a position and an orientation of the distal tip such that the distal tip remains on a target line and pointed along the target line.

Another specific example is a medical system including a vision probe that includes: a tube containing a plurality of channels including a main channel, one or more oblong channels for irrigation and suction, and one or more auxiliary channels; an imaging system extending through the main channel to a distal end of the tube; and one or more illumination fibers running through respective auxiliary channels in the tube.

The probe may a diameter less than about 2 mm. The probe may further have a keying feature shaped to engage a complementary keying feature on a catheter.

The imaging system may include: a CMOS camera mounted at the distal end of the tube; or an image fiber bundle extending through the tube to the distal end of the tube.

The tube may include an extrusion in which the channels reside.

The medical system may further include a catheter having a lumen in which the vision probe can be deployed and removed during a medical procedure. The catheter further include: a steerable segment at a distal end of the catheter; and a plurality of tendons attached to the steerable segment and extending from the steerable segment through the catheter to a proximal end of the catheter, wherein the steerable segment is actuated through pulling on proximal ends of the tendons. The steerable segment may include a tube cut to provide a plurality of flexures that permit bending of the steerable segment in pitch and yaw directions. The flexures may include Nitinol, for example.

The catheter may have a distal section with a width less than about 3 mm. A width of the probe may be more than one half a width of the catheter. A sheath may be fit within the lumen between the catheter and the vision probe, wherein the sheath is movable relative to the catheter and extendable beyond a distal end of the catheter.

Another specific example is a medical system including: a catheter having a first section and a second section, the second section adjacent to the first section; a first sensor system in the first section and configured for measurement of a pose of the first section; and a second sensor system in the second section and configured for measurement of a pose of the second section relative to the first section.

The second section may be narrower than the first section, and/or the first sensor system may be wider than the second section.

The first sensor system may include a first sensor configured to measure a position and an orientation of the first section relative to an external reference. The first sensor system may include an electromagnetic sensor that produces electrical signal that depends on a position and an orientation of the first section relative to an externally applied magnetic field, and the electromagnetic sensor may be configured for measurement of six degrees of freedom of the first section. The electromagnetic sensor may include: a first coil having a first magnetic axis; and a second coil having a second magnetic axis that is askew from the first magnetic axis. The first and second coil may be rigidly fixed to one another. The first and second coils may be within a cross-section of the first section. The first measurement system may further include an accelerometer configured to measure a direction of gravity.

The second sensor system may include a second sensor configured to measure a position and an orientation of the second section relative to the first section. The second sensor system comprises shape sensor. The shape sensor may extend along at least a portion of the first and second sections, and first and second coils in the first sensor system may positioned at separated locations along a length of the shape sensor. The shape sensor may be configured to measure a spatial relationship between the first coil and the second coil.

Another specific example is a method for sensing a pose of a distal tip of an elongated flexible structure of a medical instrument. The method includes: applying a time-varying magnetic field to a space containing at least a portion of the flexible structure; analyzing an electric signal induced in a first coil positioned at a location along the flexible structure of the medical instrument, wherein the location is separated from a proximal end and the distal tip of the flexible structure; and measuring a shape of a portion of the flexible structure of the medical instrument, wherein the portion measured extends from the location of the coil toward the distal end of the flexible section.

The flexible structure may include a catheter, and the catheter may include: a first section containing the coil; and a second section adjacent to the first section and extending to the distal tip.

The shape may be measured using a fiber shape sensor.

Analyzing the electric signal in the first coil may include determining a global position and orientation at a point along the fiber shape sensor. Determining the global position and orientation may further include using a second electrical signal induced in a second coil positioned along the flexible structure.

### BRIEF DESCRIPTION OF THE DRAWINGS

Fig. 1 shows a robotic catheter system in accordance with an embodiment of the invention having multiple control modes.
Fig. 2 shows an embodiment of a steerable segment for an actuated distal tip that can be employed in the system of Fig. 1.
Figs. 3A and 3B show cross-sectional views of proximal and distal sections of a catheter in accordance with an embodiment of the invention.
Fig. 4 shows a cross-sectional view of a vision probe that may be deployed in the catheter of Figs. 3A and 3B and swapped out for use of medical probes in the catheter shown in Figs. 3A and 3B.
Fig. 5 is a flow diagram of a process for using the catheter system with a removable vision system and multiple control modes.
Fig. 6 is a flow diagram of a catheter control process in a holding mode.
Fig. 7 shows sensing coils that can be employed in electromagnetic sensors in medical systems in some embodiments of the invention.
Figs. 8A, 8B, 8C, and 8D illustrate alternative configurations for sensor systems in accordance with embodiments of the invention including electromagnetic and shape sensors.
Fig. 9 shows cross-sections of a catheter system containing a six-degree-of-freedom EM sensor and a catheter system containing two five-degree-of-freedom EM sensors.

Use of the same reference symbols in different figures indicates similar or identical items.

### DETAILED DESCRIPTION

A catheter system can employ a vision probe that is interchangeable with one or more medical probes or tools. The vision probe can be removed and replaced with a medical probe used in a medical procedure. The interchanging of the vision and medical probes may permit the catheter system to have a smaller diameter and thus navigate smaller passages than would a similar system that simultaneously accommodates both vision and medical systems. Alternatively, interchanging probes allow more space for vision and medical systems having greater functionality than might a catheter that must simultaneously accommodate both vision and medical systems.

One method for using a catheter system includes steering a catheter along a body lumen for at least part of the path to a work site for a medical procedure. A vision system may then be deployed in the catheter during the steering and/or used to view the work site reached when navigation is complete. The vision system can also be used to help identify a desired working configuration for the catheter and when manipulating the catheter into the desired working configuration. The control system of the catheter can then record the working configuration and may be placed in a "holding" mode in which the pose of the catheter relative to a patient is monitored and the catheter is actuated to actively maintain or return to the recorded working configuration. When the vision system is removed, the catheter may still provide sensing, steering, and holding capabilities for use of a medical probe that replaces the vision probe. The division of functionality between the vision probe and the catheter provides a high level of utility within a compact cross-section. The control system may provide different types of control modes, which may be useful for different types of medical probes or different types of medical procedures. For example, if the medical probe includes a laser, a combination of the location and orientation of the distal tip of the catheter can be controlled so that the distal tip remains targeted on a specific location in the patient. An alternative holding mode can maintain the location of the distal tip of the catheter while permitting the orientation of the distal tip to change or maintain a distal tip along a line.

A robotic catheter for use in small lumens such as airways and passages in the respiratory tract may employ combinations of one or more EM sensors and a fiber shape sensor to provide accurate measurements of the pose of a small-diameter distal tip.

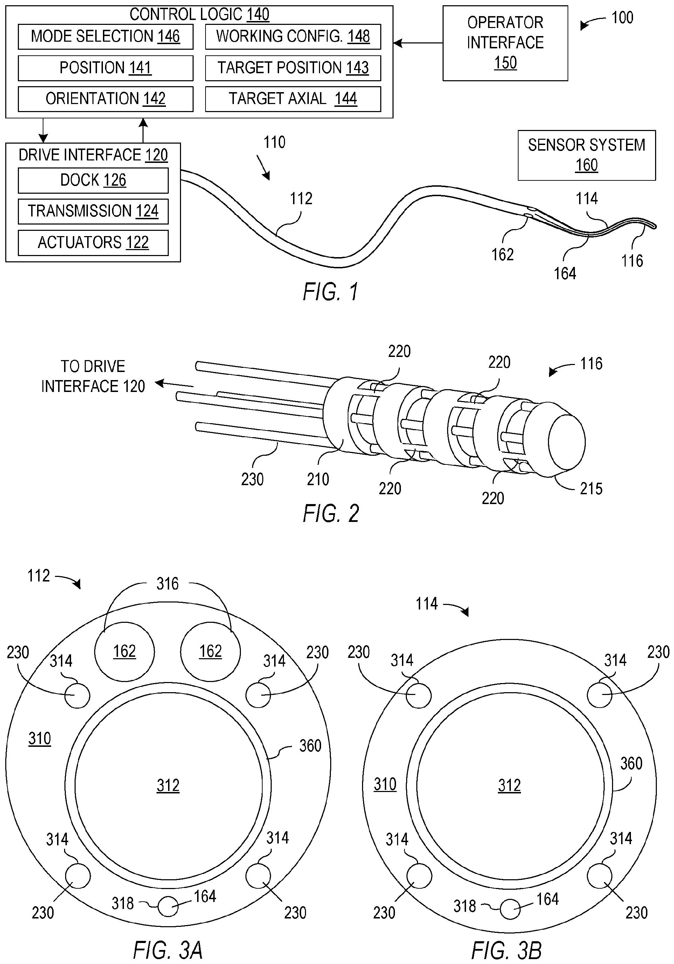

Fig. 1 schematically illustrates a catheter system 100 in accordance with one embodiment of the invention. In the illustrated embodiment, catheter system 100 includes a catheter 110, a drive interface 120, control logic 140, an operator interface 150, and a sensor system 160.

Catheter 110 is a generally flexible device having one or more lumens including a main lumen that can accommodate interchangeable probes such as described further below. Flexible catheters can be made using a braided structure such as a woven wire tube with inner or outer layers of a flexible or low friction material such as polytetrafluoroethylene (PTFE). In one embodiment, catheter 110 includes a bundle of lumens or tubes held together by a braided jacket and a reflowed (i.e., fused by melting) jacket of a material such as Polyether Block Amide (Pebax). Alternatively, an extrusion of a material such as Pebax can similarly be used to form multiple lumens in catheter 110. Catheter 110 particularly includes a main lumen for interchangeable probe systems and smaller lumens for pull wires and sensor lines. In the illustrated embodiment, catheter 110 has a proximal section 112 attached to drive interface 120 and a distal section 114 that extends from the proximal section 112. An additional steerable segment 116 (e.g., a metal structure such as shown in Fig. 2 and described further below) can form the distal subsection of distal section 114. Pull wires extend from drive system 120 through proximal section 112 and distal section 114 and connect to steerable segment 116.

The overall length of catheter 110 may be about 60 to 80 cm or longer with distal section 114 being about 15 cm long and steerable segment 116 being about 4 to 5 cm long. In accordance with an aspect of the invention, distal section 114 has a smaller diameter than does proximal section 112 and thus can navigate smaller natural lumens or passages. During a medical procedure, at least a portion of proximal section 112 and all of distal section 114 may be inserted along a natural lumen such as an airway of a patient. The smaller diameter of distal section 114 permits use of distal section 114 in lumens that may be too small for proximal section 112, but the larger diameter of distal section 114 facilitates inclusion of more or larger structures or devices such as electromagnetic (EM) sensors 162 that may not fit in distal section 114.

Steerable segment 116 is remotely controllable and particularly has a pitch and a yaw that can be controlled using pull wires. Steerable segment 116 may include all or part of distal section 114 and may be simply implemented as a multi-lumen tube of flexible material such as Pebax. In general, steerable segment 116 is more flexible than the remainder of catheter 110, which assists in isolating actuation or bending to steerable segment 116 when drive interface 120 pulls on actuating tendons. Catheter 110 can also employ additional features or structures such as use of Bowden cables for actuating tendons to prevent actuation from bending proximal section 112 (or bending any portion the section of 114 other than steerable segment 116) of catheter 110. Fig. 2 shows one specific embodiment in which steerable segment 116 is made from a tube 210 that in catheter 110 of Fig. 1 contains multiple tubes defining a main lumen for a probe system and smaller lumens for actuation tendons 230 and a shape sensor not shown in Fig. 2. In the illustrated embodiment, tendons 230 are placed 90° apart surrounding lumen 312 to facilitate steering catheter 110 in pitch and yaw directions defined by the locations of tendons 230. A reflowed jacket, which is not shown in Fig. 2 to better illustrate the internal structure of steerable segment 116, may also cover tube 210. As shown in Fig. 2, tube 210 is cut or formed to create a series of flexures 220. Tendons 230 connect to a incorporated230 can be coated or uncoated, single filament or multi strand wires, cables, Bowden cables, hypotubes, or any other structures that are able to transfer force from drive interface 120 to distal tip 215 and limit bending of proximal section 112 when drive interface 120 pulls on tendons 230. Tendons 230 can be made of any material of sufficient strength including but not limited to a metal such as steel or a polymer such as Kevlar. In operation, pulling harder on any one of tendons 230 tends to cause steerable segment 116 to bend in the direction of that tendon 230. To accommodate repeated bending, tube 210 may be made of a material such as Nitinol, which is a metal alloy that can be repeatedly bent with little or no damage.

Drive interfaces 120 of Fig. 1, which pulls on tendons 230 to actuate steerable segment 116, includes a mechanical system or transmission 124 that converts the movement of actuators 122, e.g., electric motors, into movements of (or tensions in) tendons 230 that run through catheter 110 and connect to steerable segment 116. (Push rods could conceivably be used in catheter 110 instead of tendons 230 but may not provide a desirable level of flexibility.) The movement and pose of steerable segment 116 can thus be controlled through selection of drive signals for actuators 122 in drive interface 120. In addition to manipulating tendons 230, drive interface 120 may also be able to control other movement of catheter 110 such as range of motion in an insertion direction and rotation or roll of the proximal end of catheter 110, which may also be powered through actuators 122 and transmission 124. Backend mechanisms or transmissions that are known for flexible-shaft instruments could in general be used or modified for drive interface 120. For example, some known drive systems for flexible instruments are described in U.S. Pat. App. Pub. No. 2010/0331820, entitled "Compliant Surgical Device,". Drive interface 120 in addition to actuating catheter 110 should allow removal and replacements of probes in catheter 110, so that the drive structure should be out of the way during such operations.

A dock 126 in drive interface 120 can provide a mechanical coupling between drive interface 120 and catheter 110 and link actuation tendons 230 to transmission 124. Dock 126 may additionally contain an electronic or optical system for receiving, converting, and/or relaying sensor signals from portions of sensor system 160 in catheter 110 and contain an electronic or mechanical system for identifying the probe or the type of probe deployed in catheter 110.

Control logic 140 controls the actuators in drive interface 120 to selectively pull on the tendons as needed to actuate and steer steerable segment 116. In general, control logic 140 operates in response to commands from a user, e.g., a surgeon or other medical personnel using operator interface 150, and in response to measurement signals from sensor system 160. However, in holding modes as described further below, control logic 140 operates in response to measurement signals from sensor system 160 to maintain or acquire a previously identified working configuration. Control logic 140 may be implemented using a general purpose computer with suitable software, firmware, and/or interface hardware to interpret signals from operator interface 150 and sensor system 160 and to generate control signals for drive interface 120. Details concerning control logic may be found in U.S. Pat. App. Pub. No. 2011/0282491 (disclosing "Drive Force Control in Medical Instrument Providing Position Measurements") and in U.S. Pat. App. Pub. No. 20120123441 (disclosing "Tension Control in Actuation of Multijoint Medical Instrument").

In the illustrated embodiment, control logic 140 includes multiple modules 141, 142, 143, and 144 that implement different processes for controlling the actuation of catheter 110. In particular, modules 141, 142, 143, and 144 respectively implement a position stiffening mode, an orientation stiffening mode, a target position mode, and a target axial mode, which are described further below. A module 146 selects which control process will be used and may base the selection on user input, the type or status of the probe deployed in catheter 110, and the task being performed. Control logic 140 also includes memory storing parameters 148 of a working configuration of steerable segment 116 that is desired for a task, and each of the modules 141, 142, 143, and 144 can use their different control processes to actively maintain or hold the desired working configuration.

Operator interface 150 may include standard input/output hardware such as a display, a keyboard, a mouse, a joystick, or other pointing device or similar I/O hardware that may be customized or optimized for a surgical environment. In general, operator interface 150 provides information to the user and receives instructions from the user. For example, operator interface 150 may indicate the status of system 100 and provide the user with data including images and measurements made by system 100. One type of instruction that the user may provide through operator interface 150, e.g., using a joystick or similar controller, indicates the desired movement or position of steerable segment 116, and using such input, control logic 140 can generate control signals for actuators in drive interface 120. Other instructions from the user can, for example, select an operating mode of control logic 140.

Sensor system 160 generally measures a pose of steerable segment 116. In the illustrated embodiment, sensor system 160 includes EM sensors 162 and a shape sensor 164. EM sensors 162 include one or more conductive coils that may be subjected to an externally generated electromagnetic field. Each coil of EM sensors 162 then produces an induced electrical signal having characteristics that depend on the position and orientation of the coil relative to the externally generated electromagnetic field. In an exemplary embodiment, EM sensors 162 are configured and positioned to measure six degrees of freedom, e.g., three position coordinates X, Y, and Z and three orientation angles indicating pitch, yaw, and roll of a base point. The base point in system 100 is at or near the end of proximal section 112 and the start of distal section 114 of catheter 110. Shape sensor 164 in the exemplary embodiment of the invention includes a fiber grating that permits determination of the shape of a portion of catheter 110 extending from the base point, e.g., the shape of distal section 114 or steerable segment 116. Such shape sensors using fiber gratings are further described in U.S. Pat. No. 7,720,322, entitled "Fiber Optic Shape Sensor,". An advantage of the illustrated type of sensor system 160 is that EM sensors 162 can provide measurements relative to the externally generated magnetic field, which can be calibrated relative to a patient's body. Thus, system 160 can use EM sensors 162 to reliably measure the position and orientation of a base point for shape sensor 164, and shape sensor 164 need only provide shape measurement for a relatively short distance. Additionally, distal section 114 only contains shape sensor 164 and may have a diameter that is smaller than the diameter of proximal section 112. More generally, sensor system 160 need only be able to measure the pose of steerable segment 116, and other types of sensors could be employed.

Figs. 3A and 3B respectively show cross-sections of the proximal and distal sections 112 and 114 of catheter 110 in one embodiment of the invention. Fig. 3A shows an embodiment of catheter 110 having a body 310 that includes a main lumen 312 for a vision or medical probe, lumens 314 containing tendons 230, lumens 316 containing EM sensors 162 or associated signal wires, and a lumen 318 containing a fiber shape sensor 164. Main lumen 312, tendon lumens 314, and a shape sensor lumen 318 extend into distal section 114 as shown in Fig. 3B, but lumens 316 for EM sensors 162 are not needed in distal section 114 because EM sensors 162 are only in proximal section 112. Accordingly, distal section 114 can be smaller than proximal section 112 particularly because the lumen 318 for fiber shape sensor 164 fits between two lumens 314 for pull wires and does not negatively affect the outside diameter of distal section 114. In an exemplary embodiment, body 310 in proximal section 112 has an outer diameter of about 4 mm (e.g., in a range from 3 to 6 mm) and provides main lumen 312 with a diameter of about 2 mm (e.g., in a range from 1 to 3 mm) and in distal section 114 has an outer diameter of about 3 mm (e.g., in a range from 2 to 4 mm) while maintaining the diameter of main lumen 312 at about 2 mm. A smooth taper (as shown in Fig. 1) or an abrupt step in body 310 can be used at the transition from the larger diameter of proximal section 112 to the smaller diameter of distal section 114.

The specific dimensions described in above are primarily for a catheter that accommodates probes having a diameter of 2 mm, which is a standard size for existing medical tools such as lung biopsy probes. However, alternative embodiments of the invention could be made larger or smaller to accommodate medical probes with a larger or smaller diameter, e.g., 1 mm diameter probes. A particular advantage of such embodiments is that a high level of functionality is provided in a catheter with relative small outer diameter when compared to the size of probe used in the catheter.

Figs. 3A and 3B also show a sheath 360 that may be employed between catheter body 310 and a probe in main lumen 312. In one embodiment of catheter 110, sheath 360 is movable relative to body 310 and can be extended beyond the end of steerable segment 116. This may be advantageous in some medical procedures because sheath 360 is even smaller than distal section 114 and therefore may fit into smaller natural lumens or passages. For example, if catheter 110 reaches a branching of lumens that are too small to accommodate steerable segment 116, steerable segment 116 may be pointed in the direction of the desired branch, so that sheath 360 can be pushed beyond the end of steerable segment 116 and into that branch. Sheath 360 could thus reliably guide a medical probe into the desired branch. However, sheath 360 is passive in that it is not directly actuated or steerable. In contrast, distal section 114 accommodates actuation tendons 230 that connect to steerable segment 116 and can be manipulated to steer or pose steerable segment 116. In some medical applications, the active control of steerable segment 116 is desirable or necessary during a medical procedure, and passive sheath 360 may not be used in some embodiments of the invention.

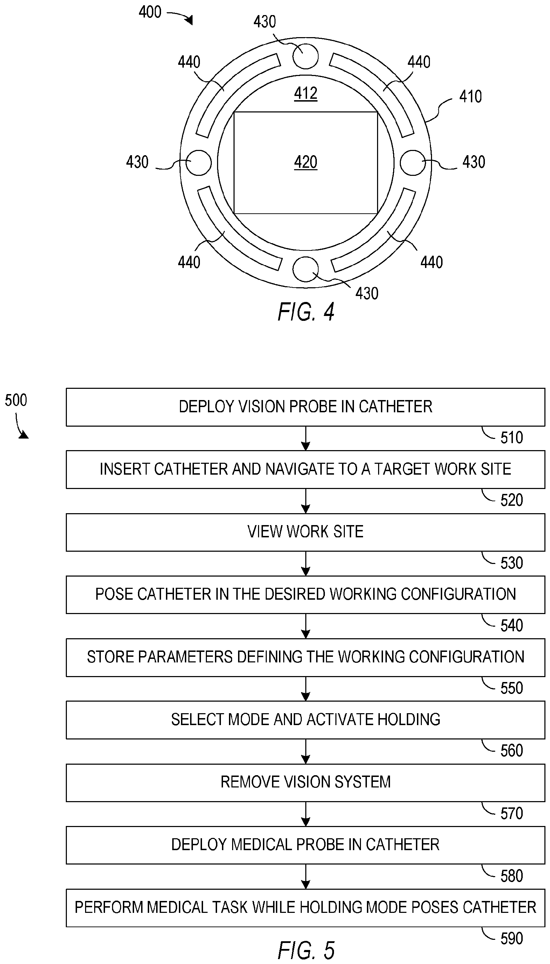

Main lumen 312 is sized to accommodate a variety of medical probes. One specific probe is a vision probe 400 such as illustrated in Fig. 4. Vision probe 400 has a flexible body 410 with an outer diameter (e.g., about 2 mm) that fits within the main lumen of catheter 110 and with multiple inner lumens that contain the structures of vision probe 400. Body 410 may be formed using an extruded flexible material such as Pebax or another polymer, which allows creation of multiple lumens and thin walls for maximal utility in minimal cross-sectional area. A multi-lumen extrusion also neatly organizes the location of the components. The length of body 410 may optionally include a combination of two multi-lumen extrusions, for example, a distal extrusion "butt-welded" to a proximal extrusion. This may be done, for example, so that the proximal or distal extrusion has desired shape, e.g., a clover-leaf or oval outside shape, to mate with a complementary keying feature in catheter 110. These mating shapes or keying structures can prevent the probe from rotating within the catheter and assure a known orientation of camera 320 relative to catheter 110.

In the illustrated embodiment, the structure of vision probe 400 includes a CMOS camera 420, which is at the distal end of the probe and connected through one or more signal wires (not shown) that extend along the length of vision probe 400, e.g., to provide a video signal to control logic 140 or operator interface 150 as shown in Fig. 1. Alternatively, a fiber bundle imaging system could be employed, but CMOS cameras 420 can typically provide images of higher quality than can be achieved with fiber bundle imaging systems. Vision probe 400 also includes illumination fibers 430 that surround camera 420 and provide light for imaging within a body lumen. In an exemplary embodiment, illumination fibers 430 are made of a flexible material such as plastic, which tends to be more flexible than glass fibers. Oblong fluid ports 440 are provided in body 410 for suction and irrigation that may be useful, for example, for rinsing of a lens of camera 420. Fluid ports 440 can also be used for delivering drugs, e.g., for numbing, before vision probe 400 is removed from catheter 110 and replaced with a biopsy probe. Although the illustrated embodiment of vision probe 400 includes multiple fluid ports 440, a single fluid port could be used for both irrigation and suction, and vision probe 400 could alternatively have only a single fluid port to save space. Vision probe 400 may additionally include an electromagnetic sensor (not shown) embedded just proximally to CMOS camera 420 to provide additional pose information about the tip of vision probe 400.

Vision probe 400 is adapted to be inserted or removed from catheter 110 while catheter 110 is in use for a medical procedure. Accordingly, vision probe 400 is generally free to move relative to catheter 110. While movement relative to catheter 110 is necessary or desirable during insertion or removal of vision probe 400, the orientation of a vision probe 400 (and some medical probes) may need to be known for optimal or easier use. For example, a user viewing video from vision probe 400 and operating a controller similar to a joystick to steer catheter 110 generally expects the directions of movement of the controller to correspond to the response of steerable segment 116 and the resulting change in the image from vision probe 400. Operator interface 150 needs (or at least can use) information on the orientation of vision probe 400 relative to tendons 230 in order to provide a consistency in directions used in the user interface. In accordance with an aspect of the invention, a keying system (not shown) can fix vision probe 400 into a known orientation relative to catheter 110 and tendons 230. The keying system may, for example, be implemented through the shape of a proximal or distal section of probe 400 or include a spring, fixed protrusion, or latch on vision probe 400 or steerable segment 116 and a complementary notch or feature in steerable segment 116 or vision probe 400.

Vision probe 400 is only one example of a probe system that may be deployed in catheter 110 or guided through catheter 110 to a work site. Other probe systems that may be used include, but are not limited to, biopsy forceps, biopsy needles, biopsy brushes, ablation lasers, and radial ultrasound probes. In general, catheter 110 can be used with existing manual medical probes that are commercially available from medical companies such as Olympus Europa Holding GmbH.

The catheter system 100 of Fig. 1 can be used in procedures that swap a vision probe and a medical probe. Fig. 5 is a flow diagram of one embodiment of a process 500 for using the catheter system 100 of Fig. 1. In process 500, vision probe 400 is deployed in catheter 110 in step 510, and catheter 110 is inserted along a path including a natural lumen of a patient. For example, for a lung biopsy, steerable segment 116 of catheter 110 may be introduced through the mouth of a patient into the respiratory tract of the patient. Vision probe 400 when fully deployed in catheter 110 may fit into a keying structure that keeps vision probe 400 in a desired orientation at or even extending beyond steerable segment 116 to provide a good forward view from steerable segment 116 of catheter 110. As noted above, steerable segment 116 of catheter 110 is steerable, and vision probe 320 can provide video of the respiratory tract that helps a user when navigating catheter 110 toward a target work site. However, use of vision probe 400 during navigation is not strictly necessary since navigation of catheter 110 may be possible using measurements of sensor system 160 or some other system with or without vision probe 400 being deployed or used in catheter 110. The path followed to the work site may be entirely within natural lumens such as the airways of the respiratory track or may pierce and pass through tissue at one or more points.

When steerable segment 116 reaches the target work site, vision probe 400 can be used to view the work site as in step 530 and to pose steerable segment 116 for performance of a task at the target work site as in step 540. Posing of steerable segment 116 may use images or visual information from vision probe 400 and measurements from sensor system 160 to characterize the work site and determine the desired working configuration. The desired working configuration may also depend on the type of tool that will be used or the next medical task. For example, reaching a desired working configuration of catheter 110 may bring the distal tip of steerable segment 116 into contact with tissue to be treated, sampled, or removed with a medical tool that replaces vision probe 400 in catheter 110. Another type of working configuration may point steerable segment 116 at target tissue to be removed using an ablation laser. For example, tissue could be targeted in one or more 2D camera views while vision probe 400 is still in place in catheter 110, or target tissue can be located on a virtual view of the work site using preoperative 3D imaging data together with the position sensing relative to patient anatomy. Still another type of working configuration may define a line for the insertion of a needle or other medical tool into tissue, and the working configuration includes poses in which the distal tip of steerable segment 116 is along the target line. In general, the desired working configuration defines constraints on the position or the orientation of the distal tip of steerable segment 116, and the shape of more proximal sections of catheter 110 is not similarly constrained and may vary as necessary to accommodate the patient.

Step 550 stores in memory of the control logic parameters that identify the desired working configuration. For example, the position of a distal tip or target tissue can be defined using three coordinates. A target line for a need can be defined using the coordinates of a point on the line and angles indicating the direction of the line from that point. In general, control logic 120 uses the stored parameters that define the desired working configuration when operating in a holding mode that maintains steerable segment 116 of catheter 110 in the desired working configuration as described further below.

Step 560 selects and activates a holding mode of the catheter system after the desired working configuration has been established and recorded. Control logic 140 for catheter 110 of Fig. 1 may have one or more modules 141, 142, 143, and 144 implementing multiple stiffening modes that may be used as holding modes when the desired configuration of steerable segment 116 has fixed constraints. The available control modes may include one or more of the following.
1.) A position stiffness mode compares the position of the distal tip of steerable segment 116 as measured by sensor system 160 to a desired tip position and controls the actuators to minimize the difference in desired and measured tip positions. The position stiffness mode may particularly be suitable for general manipulation tasks in which the user tries to precisely control the position of the tip and for situations where the distal tip contacts tissue.
2.) An orientation stiffness mode compares the measured orientation or pointing direction of the distal tip to a desired pointing direction of the distal tip and controls the actuators to minimize the difference in desired and actual tip pointing direction. This orientation stiffening that may be suitable, e.g., when controlling an imaging device such as vision probe 400 attached steerable segment 116, in which case the viewing direction is kept as desired, while the exact position of steerable segment 116 may be less important.
3.) A target position stiffness mode uses a combination of the measured tip position and pointing direction to control catheter 110 to always point the distal tip of steerable segment 116 towards a specified target point some distance in front of steerable segment 116. In case of external disturbances, control logic 140 may control the actuators to implement this target position stiffening behavior, which may be suitable, e.g., when a medical probe inserted though the catheter contains an ablation laser that should always be aimed at a target ablation point in tissue.
4.) A target axial motion stiffness mode uses a combination of the measured tip position and pointing direction to ensure that the distal tip of steerable segment 116 is always on a line in space and has a pointing direction that is also along that line. This mode can be useful, e.g., when inserting a biopsy needle along a specified line into tissue. Tissue reaction forces could cause the flexible section of catheter 110 to bend while inserting the needle, but this control strategy would ensure that the needle is always along the right line.

The selection of a mode in step 560 could be made through manual selection by the user, based on the type of probe that is being used (e.g., grasper, camera, laser, or needle) in catheter 110, or based on the activity catheter 110 is performing. For example, when a laser is deployed in catheter 110, control logic 120 may operate in position stiffness mode when the laser deployed in catheter 110 is off and operate in target position stiffness mode to focus the laser on a desired target when the laser is on. When "holding" is activated, control logic 140 uses the stored parameters of the working configuration (instead of immediate input from operator interface 150) in generating control signals for drive interface 120.

The vision probe is removed from the catheter in step 570, which clears the main lumen of catheter 110 for the step 580 of inserting a medical probe or tool through catheter 110. For the specific step order shown in Fig. 5, control logic 140 operates in holding mode and maintains steerable segment 116 in the desired working configuration while the vision system is removed (step 570) and the medical probe is inserted (step 580). Accordingly, when the medical probe is fully deployed, e.g., reaches the end of steerable segment 116, the medical probe will be in the desired working configuration, and performance of the medical task as in step 590 can be then performed without further need or use of the removed vision probe. Once the medical task is completed, the catheter can be taken out of holding mode or otherwise relaxed so that the medical probe can be removed. The catheter can then be removed from the patient if the medical procedure is complete, or the vision or another probe can be inserted through the catheter if further medical tasks are desired.

In one alternative for the step order of process 500, catheter 110 may not be in a holding mode while the medical probe is inserted but can be switched to holding mode after the medical probe is fully deployed. For example, catheter 110 may be relaxed or straightened for easy remove of vision probe 400 (step 570) and insertion of the medical probe (step 580). Once holding mode is initiated, e.g., after insertion of the medical probe, control logic 140 will control the drive interface 130 to return steerable segment 116 to the desired working configuration if steerable segment 116 has moved since being posed in the desired working configuration. Thereafter, control logic 140 monitors the pose of steerable segment 116 and actively maintains steerable segment 116 in the desired working configuration while the medical task is performed in step 590.

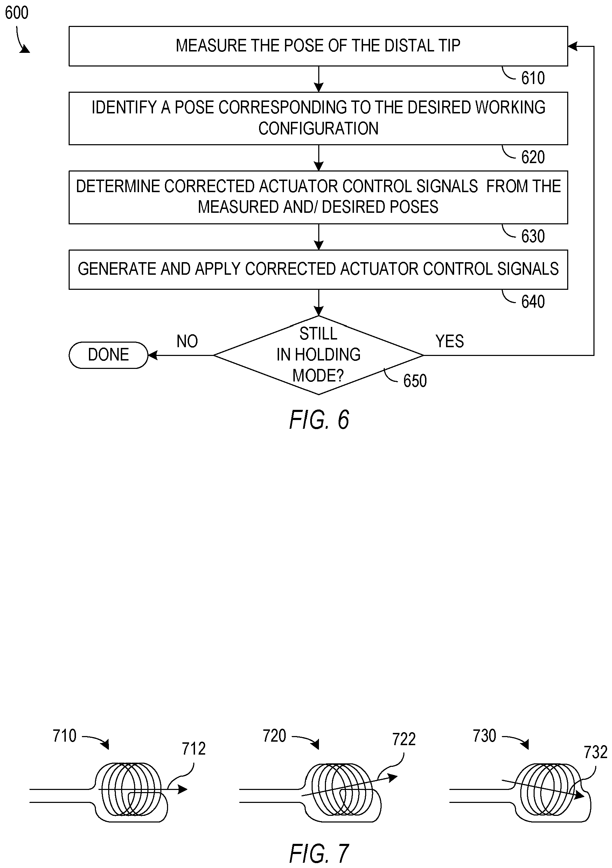

Fig. 6 shows a flow diagram of a process 600 of a holding mode that can be implemented in control logic 140 of Fig. 1. Process 600 begins in step 610 with receipt of measurement signals from sensor system 160. The particular measurements required depend on the type of holding mode being implemented, but as an example, the measurements can indicate position coordinates, e.g., rectangular coordinates X, Y, and Z, of the distal tip of steerable segment 116 and orientation angles, e.g., angles θ_{X}, θ_{Y}, and θ_{Z} of a center axis of the distal tip of steerable segment 116 relative to coordinate axes X, Y, and Z. Other coordinate systems and methods for representing the pose of steerable segment 116 could be used, and measurements of all coordinates and direction angles may not be necessary. However, in an exemplary embodiment, sensor system 160 is capable of measuring six degrees of freedom (DoF) of the distal tip of steerable segment 116 and of providing those measurements to control logic 140 in step 610.

Control logic 140 in step 620 determines a desired pose of steerable segment 116. For example, control logic 140 can determine desired position coordinates, e.g., X', Y', and Z', of the end of steerable segment 116 and desired orientation angles, e.g., angles θ'_{X}, θ'_{Y}, and θ'_{Z} of the center axis of steerable segment 116 relative to coordinate axes X, Y, and Z. The holding modes described above generally provide fewer than six constraints on the desired coordinates. For example, position stiffness operates to constrain three degrees of freedom, the position of the end of steerable segment 116 but not the orientation angles. In contrast, orientation stiffness mode constrains one or more orientation angles but not the position of end of steerable segment 116. Target position stiffness mode constrains four degrees of freedom, and axial stiffness mode constrains five degrees of freedom. Control logic 610 can impose further constraints to select one of set of parameters, e.g., X', Y', and Z' and angles θ'_{X}, θ'_{Y}, and 0'z, that provides the desired working configuration. Such further constraints include but are not limited to mechanical constraints required by the capabilities of steerable segment 116 and of catheter 110 generally and utilitarian constraints such as minimizing movement of steerable segment 116 or providing desired operating characteristics such as smooth, non-oscillating, and predictable movement with controlled stress in catheter 110. Step 620 possibly includes just keeping a set pose steerable segment 116 by finding smallest movement from the measured pose to a pose satisfying the constraints, e.g., finding the point on the target line closest to the measure position for axial motion stiffness or finding some suitable pose from registered pre-op data that is close to the current pose.

Control logic 140 in step 630 uses the desired and/or measured poses to determine corrected control signals that will cause drive interface 120 to move steerable segment 116 to the desired pose. For example, the mechanics of catheter 110 and drive interface 120 may permit development of mappings from the desired coordinates X', Y', and Z' and angles θ'_{X}, θ'_{Y}, and θ'_{Z} to actuator control signals that provide the desired pose. Other embodiments may use differences between the measured and desired pose to determine corrected control signals. In general, the control signals may be used not only to control actuators connected through tendons to steerable segment 116 but may also control (to some degree) insertion or roll of catheter 110 as a whole.

A branch step 650 completes a feedback loop by causing process 600 to return to measurement step 610 after control system 140 applies new control signals drive interface 120. The pose of distal tip is thus actively monitored and controlled according to fixed constraints as long as control system 120 remains in the holding mode. It may be noted, however, that some degrees of freedom of steerable segment 116 may not require active control. For example, in orientation stiffness mode, feedback control could actively maintain pitch and yaw of steerable segment 116, while the mechanical torsional stiffness of catheter 110 is relied on hold the roll angle fixed. However, catheter 110 in general may be subject to unpredictable external forces or patient movement that would otherwise cause catheter 110 to move relative to the work site, and active control as in process 600 is needed to maintain or hold the desired working configuration.

The sensor system 160 of a catheter 100 as noted above can employ both an EM sensor 162 and a fiber shape sensor 164. EM sensors or trackers are state-of-the-art position and orientation sensors that combine high global accuracy with small package size (e.g., about 1x10mm). EM sensors are commercially available from companies such as Ascension Technology Corporation and Northern Digital Inc. Shape sensing technology, which may be used in the above described embodiments, commonly employ reflections and interference within an optical fiber to measure the shape along the length of the optical fiber. This shape sensing technology is good for giving 6-DoF relative measurements between two points along the fiber as well as measuring bend angles of controllable joints or providing full three-dimensional shape information. A typical fiber shape sensor of this type may have a diameter of about 0.2 mm, which is considerably smaller than a typical EM sensor.

Fig. 7 illustrates three different types of sensing coils 710, 720, and 730 that could be used in an EM sensor. In operation, the sensing coil, e.g., coil 710, in the catheter or other device is placed in a well-controlled magnetic field that an external EM generator produces. The EM generator typically has the form of a square or cylindrical box of 20-60 cm wide and several cm thick and may have a fixed position relative to the patient. The magnetic field produced by the generator varies in time and induces a voltage and electric current in the sensing coil 710. U.S. Pat. No. 7,197,354, entitled "System for Determining the Position and Orientation of a Catheter"; U.S. Pat. No. 6,833,814, entitled "Intrabody Navigation System for Medical Applications"; and U.S. Pat. No. 6,188,355, entitled "Wireless Six-Degree-of-Freedom Locator" describe the operation of some EM sensor systems suitable for in medical environment and are hereby. U.S. Pat. No. 7,398,116, entitled "Methods, Apparatuses, and Systems useful in Conducting Image Guided Interventions," U.S. Pat. No. 7,920,909, entitled "Apparatus and Method for Automatic Image Guided Accuracy Verification," U.S. Pat. No. 7,853,307, entitled "Methods, Apparatuses, and Systems Useful in Conducting Image Guided Interventions," and U.S. Pat. No. 7,962,193, entitled "Apparatus and Method for Image Guided Accuracy Verification" further describe systems and methods that can use electromagnetic sensing coils in guiding medical procedures. In general, the induced voltage in a sensing coil depends on time derivative the magnetic flux, which in turn depends on the strength of the magnetic field and the direction of the magnetic field relative to a normal to the areas of loops in the coil. The field generator can vary the direction and magnitude of the magnetic field in a systematic manner that enables at least partial determination of the pose of coil 710 from the induced electric signal. Up to five degrees of freedom can be determined using a sensor 162 containing a single sensing coil 710. However, sensing coil 710 is cylindrically symmetric, so that a roll angle, i.e., an angle indicating orientation about a normal 712 to the inductive areas of coil 710, cannot be determined. Only the position and the pointing direction can be determined using a single coil 710. Even so, a 5-Degree-of-Freedom (5-DoF) sensor containing a single sensing coil 710 is useful in many medical systems. In particular, the mechanical shape of a typical sensing coil (long and slender) fits well with the mechanical shape of minimally invasive medical tools, and if the tool is rotationally symmetrical (e.g. in the case of a needle or laser fiber), the roll angle is not relevant.

A robotic control catheter such as catheter 110 may need a 6-DoF measurement including a measurement of the roll angle so that the positions of actuating tendons are known. If measurement of the roll angle is of interest, two 5-DoF EM sensors can be combined to create a 6-DoF EM sensor. One specific configuration of a 6-DoF EM sensor uses two coils such as 710 with the inductive areas of two coils having normal vectors that are askew, e.g., perpendicular to each other. More generally, the two coils need to be arranged so that the normal vectors to inductive areas are not along the same axis, and larger angles between the normal vectors generally provide better measurement accuracy. Coils 720 and 730 illustrate how a coil 720 or 730 that may have wire loops with a normal 722 or 732 that is at a non-zero angle to the axes of a cylinder containing the coil 720 or 730. Coils 720 and 730 can thus be oriented along the same direction, e.g., along the length of a catheter or other medical tool, and still be used to measure six degrees of freedom.

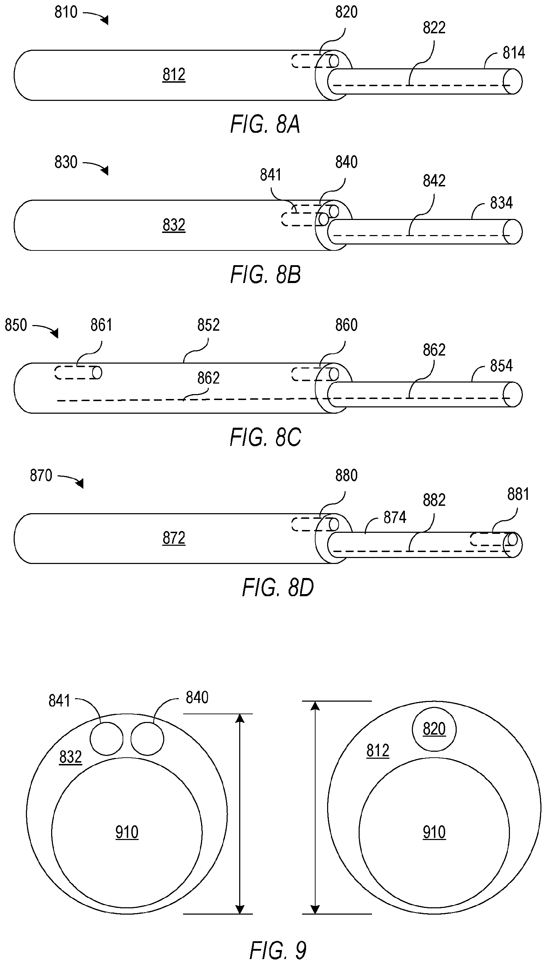

Fig. 8A shows a configuration of a catheter system 810 having a proximal section 812 containing a 6-DoF EM sensor 820 and a distal section 814 containing a fiber shape sensor 822. EM sensor 820 terminates at or near a distal end of proximal section 812. Accordingly, distal section 814 can have a diameter (e.g., about 3 mm to accommodate a probe diameter of about 2 mm) that is smaller than the diameter (e.g., about 4 mm) of proximal section 812 because EM sensor 820 does not extend into distal section 814. The pose of distal tip of section 814 can be determined using EM sensor 820 to measure or determine the global position and orientation of a point along shape sensor 822 and using shape sensor 822 to determine the shape of distal section 814 extending from the measured point. The accuracy of shape sensor 822 can be relatively high because shape sensor 822 only needs to measure the shape of a relatively short section 814 of catheter 810, rather than the entire length of catheter 810. For example, in one case, the accuracy of the position measurement for the distal tip of section 814 is a function of the position and orientation accuracy of the EM sensor 820 (typically about 1 mm and .01 radians respectively) and the position accuracy of the shape sensor (.15% of the length of section 814). If 6-DoF EM sensor 820 is about 115 mm away from the distal tip, the typical tip position accuracy would be about 2.5 mm.

Fig. 8B shows a catheter 830 that uses two 5-DoF EM sensors 840 and 841 in a proximal section 832 to measure six degrees of freedom of a base point along a shape sensor 842 that extends into a distal section 834 of catheter 830. Coils of EM sensors 840 and 841 are within the same cross-section of proximal section 832 and therefore are rigidly fixed relative to each other. EM sensors 840 and 841 can also contain sensing coils such as coils 720 and 730 having wire loops with different orientations to measure different degrees of freedom of a point along shape sensor 842. The roll angle can thus be determined using the two measured pointing directions of sensors 840 and 841 to define a reference frame. The use of 5-DoF sensors 840 and 841 may allow a reduction in the diameter of proximal section 832. In particular, 6-DoF EM sensors that are available commercially generally have diameters that are larger than the diameters of similar 5-DoF EM sensors. In accordance with an aspect of the current invention, the diameter of a catheter may be decreased through use of 5-DoF EM sensors. Fig. 9, for example, illustrates how a distal section 832 of catheter 830 can accommodate two 5-DoF EM sensors 840 and 841 and a main lumen 910 within a circular cross-sectional area that is smaller than the area of distal section 812 of catheter 810. In particular, section 812 is larger because section 812 must accommodate the main lumen and a 6-DoF sensor that has a larger diameter than do 5-DoF sensors 840 and 841.

Fig. 8C shows an embodiment of the invention using a sensor system that may allow a proximal section 852 of catheter 850 to be even smaller by using two 5-DoF EM sensors 860 and 861 that are separated along the length of the proximal section 852. Accordingly, only one 5-DoF EM sensor 860 or 861 needs to be accommodated within the cross-section of proximal section 852. However, since distal section 852 is flexible and may be bent when in use, EM sensors 860 and 861 are not rigidly fixed relative to each other, and a shape sensor 862 is used to measure the shape of the portion of proximal section 852 between EM sensors 861 and 860 and the relative orientation of EM sensors 860 and 861. The shape measurement between EM sensors 861 and 860 indicates the position and orientation of sensor 860 relative to sensor 861, and the relative configuration is needed for determination of a 6-DoF measurement from the two 5-DoF measurement. Shape sensor 862 also measures the shape of distal section 854, which indicates the position and orientation of the distal tip relative to the global position and orientation measurements determined using EM sensors 860 and 861.

Fig. 8D shows yet another catheter 870 using two 5-DoF EM sensors 880 and 881 that are separate along the length of catheter 870. The sensing system in catheter 870 of Fig. 8D differs from the sensing systems of Figs. 8A, 8B, and 8C in that one EM sensor 880 is located in a proximal section 872 of catheter 870 and the other EM sensor 881 is located in a distal section 874 of catheter 870. Accordingly, distal section 874 must be large enough to include sensor 881, but still may allow a reduction in the diameter of catheter 870 when compared to a catheter having a 6-DoF EM sensor at a distal tip.

The use of two 5-DoF EM sensors in embodiments of Figs. 8B, 8C, and 8D provides more information than is strictly required for a 6-DoF measurement. In accordance with a further aspect of the invention, one of the two 5-DoF EM sensors in catheter 830, 850, or 870 of Fig. 8B, 8C, or 8D could be replaced with another type of sensor that may not measure five degrees of freedom. For example, an accelerometer could be employed in place of one of the two EM sensors and provide a measurement of the direction of gravity, i.e., down. Provided that the symmetry axis of the 5-DoF sensor is not vertical, the combination of the measurements of a 5-DoF sensor and a measurement of the orientation relative to the vertical direction is sufficient to indicate measurements for six degrees of freedom.

Some embodiments or elements described above can be implemented in a computer-readable media, e.g., a non-transient media, such as an optical or magnetic disk, a memory card, or other solid state storage containing instructions that a computing device can execute to perform specific processes that are described herein. Such media may further be or be contained in a server or other device connected to a network such as the Internet that provides for the downloading of data and executable instructions.

Although the invention has been described with reference to particular embodiments, the description is only an example of the invention's application and should not be taken as a limitation. Various adaptations and combinations of features of the embodiments disclosed are within the scope of the invention as defined by the following claims.

## Claims

1. A medical system comprising:
a catheter (110) having a main lumen (312) and a distal tip (215) wherein the main lumen (312) accommodates either one of an imaging device (400) and a medical probe for performing a medical procedure, wherein the main lumen (312) is too narrow to simultaneously guide both the imaging device (400) and the medical probe;
a mechanical system (124) connected to control a pose of the distal tip (215) of the catheter (110);
a sensor system (160) configured to measure the pose of the distal tip (215); and
a control system (140) coupled to operate the mechanical system, wherein the control system and the sensor system (160) form a closed-loop system for monitoring and operating the distal tip (215), wherein the control system is configured to store parameters that identify a desired working configuration of the distal tip (215) while the imaging device (400) is deployed in the main lumen (312) and to hold or return the distal tip (215) of the catheter to the desired working configuration when the imaging device (400) has been removed from the catheter (110) and the medical probe is deployed in the main lumen (312),
**characterised in that**
wherein the control system (14) stores a plurality of modes (141, 142, 143, 144) for holding or returning the catheter (110) to the desired working configuration,
the control system further comprises a selection module (146) configured to select and activate one of the plurality of modes (141, 142, 143, 144) for holding or returning the catheter based on a manual selection, a type of mechanical probe, or a type of medical activity.

2. The system of claim 1, wherein a distal section of the catheter has a width less than about 3 mm.

3. The system of claim 2, wherein the main lumen (312) in the catheter has a width that is more than one half of the width of the distal section.

4. The system of claim 1, wherein the plurality of modes includes a position mode in which the control system operates the mechanical system to hold or return the distal tip (215) to a target position.

5. The system of claim 1, wherein the plurality of modes includes an orientation mode in which the control system operates the mechanical system to hold or return the distal tip (215) at a target orientation.

6. The system of claim 1, wherein the plurality of modes includes a target mode in which the control system operates the mechanical system to control a combination of a position and an orientation of the distal tip (215) to point the distal tip (215) at a target position.

7. The system of claim 1, wherein the plurality of modes includes a target axial mode in which the control system operates the mechanical system to control a combination of a position and an orientation of the distal tip (215) such that the distal tip (215) remains on or returns to a target line.

8. The system of claim 1, further comprising:
an interface configured to identify whether the imaging device (400) or the medical probe is deployed in the catheter.

9. The system of claim 8, where the interface is a user-input device that allows an operator to select a desired operating mode.

10. The system of claim 8, where the interface is configured to detect a type of medical probe deployed, and the selection module activates one of the plurality of modes depending on the detected type of medical probe.

11. The system of claim 1, further comprising the imaging device (400) that is removable from the catheter, wherein the imaging device (400) fills the main lumen (312) when the imaging device (400) is deployed in the catheter.

## Patentansprüche

1. Medizinisches System, das Folgendes umfasst:
einen Katheter (110) mit einem Hauptlumen (312) und einer distalen Spitze (215), wobei das Hauptlumen (312) entweder eine Bildgebungsvorrichtung (400) oder eine medizinische Sonde zum Durchführen eines medizinischen Verfahrens aufnimmt, wobei das Hauptlumen (312) zu schmal ist, um gleichzeitig sowohl die Bildgebungsvorrichtung (400) als auch die medizinische Sonde zu führen;
ein mechanisches System (124), das zum Steuern einer Lage der distalen Spitze (215) des Katheters (110) angeschlossen ist;
ein Sensorsystem (160), das so konfiguriert ist, dass es die Lage der distalen Spitze (215) erfasst; und
ein Steuersystem (140), das zum Betreiben des mechanischen Systems angeschlossen ist, wobei das Steuersystem und das Sensorsystem (160) einen geschlossenen Regelkreis zum Überwachen und Betreiben der distalen Spitze (215) bilden, wobei das Steuersystem so konfiguriert ist, dass es Parameter speichert, die eine gewünschte Arbeitskonfiguration der distalen Spitze (215) identifizieren, während die Bildgebungsvorrichtung (400) im Hauptlumen (312) eingesetzt ist, und die distale Spitze (215) des Katheters festhält oder in die gewünschte Arbeitskonfiguration zurückführt, wenn die Bildgebungsvorrichtung (400) aus dem Katheter (110) entfernt wurde und die medizinische Sonde im Hauptlumen (312) eingesetzt ist,
**dadurch gekennzeichnet, dass**
wobei das Steuersystem (14) eine Mehrzahl von Modi (141, 142, 143, 144) zum Festhalten oder Zurückführen des Katheters (110) in die gewünschte Arbeitskonfiguration speichert,
das Steuersystem ferner ein Auswahlmodul (146) umfasst, das so konfiguriert ist, dass es einen der Mehrzahl von Modi (141, 142, 143, 144) zum Festhalten oder Zurückführen des Katheters basierend auf einer manuellen Auswahl, einem mechanischen Sondentyp oder einer Art von medizinischer Aktivität auswählt und aktiviert.

2. System nach Anspruch 1, wobei ein distaler Abschnitt des Katheters eine Breite von weniger als etwa 3 mm hat.

3. System nach Anspruch 2, wobei das Hauptlumen (312) im Katheter eine Breite hat, die mehr als eine Hälfte der Breite des distalen Abschnitts beträgt.

4. System nach Anspruch 1, wobei die Mehrzahl von Modi einen Positionsmodus beinhaltet, in dem das Steuersystem das mechanische System betreibt, um die distale Spitze (215) festzuhalten oder zu einer Zielposition zurückzuführen.

5. System nach Anspruch 1, wobei die Mehrzahl von Modi einen Orientierungsmodus beinhaltet, in dem das Steuersystem das mechanische System betreibt, um die distale Spitze (215) festzuhalten oder zu einer Zielorientierung zurückzuführen.

6. System nach Anspruch 1, wobei die Mehrzahl von Modi einen Zielmodus beinhaltet, in dem das Steuersystem das mechanische System betreibt, um eine Kombination aus einer Position und einer Orientierung der distalen Spitze (215) zu steuern, um die distale Spitze (215) auf eine Zielposition zu richten.

7. System nach Anspruch 1, wobei die Mehrzahl von Modi einen axialen Zielmodus beinhaltet, in dem das Steuersystem das mechanische System betreibt, um eine Kombination aus einer Position und einer Orientierung der distalen Spitze (215) so zu steuern, dass die distale Spitze (215) auf einer Ziellinie bleibt oder zu ihr zurückkehrt.

8. System nach Anspruch 1, das ferner Folgendes umfasst:
eine Schnittstelle, die so konfiguriert ist, dass sie erkennt, ob die Bildgebungsvorrichtung (400) oder die medizinische Sonde im Katheter eingesetzt ist.

9. System nach Anspruch 8, wobei die Schnittstelle eine Benutzereingabevorrichtung ist, die es zulässt, dass ein Bediener einen gewünschten Betriebsmodus auswählt.

10. System nach Anspruch 8, wobei die Schnittstelle so konfiguriert ist, dass sie einen eingesetzten medizinischen Sondentyp ermittelt, und das Auswahlmodul je nach dem ermittelten medizinischen Sondentyp einen aus der Mehrzahl von Modi aktiviert.

11. System nach Anspruch 1, das ferner die Bildgebungsvorrichtung (400) umfasst, die aus dem Katheter entfernbar ist, wobei die Bildgebungsvorrichtung (400) das Hauptlumen (312) ausfüllt, wenn die Bildgebungsvorrichtung (400) in dem Katheter eingesetzt ist.

## Revendications

1. Système médical comprenant :
un cathéter (110) comportant une lumière principale (312) et une pointe distale (215), dans lequel la lumière principale (312) reçoit l'un ou l'autre d'un dispositif d'imagerie (400) et d'une sonde médicale pour l'exécution d'une procédure médicale, dans lequel la lumière principale (312) est trop étroite pour guider simultanément le dispositif d'imagerie (400) et la sonde médicale ;
un système mécanique (124) raccordé pour contrôler une pose de la pointe distale (215) du cathéter (110) ;
un système de détection (160) configuré pour mesurer la pose de la pointe distale (215) ; et
un système de commande (140) couplé pour actionner le système mécanique, le système de commande et le système de détection (160) formant un système en circuit fermé pour surveiller et actionner la pointe distale (215), le système de commande étant configuré pour la mémorisation de paramètres qui identifient une configuration de travail souhaitée de la pointe distale (215) tandis que le dispositif d'imagerie (400) est déployé dans la lumière principale (312) et pour maintenir ou renvoyer la pointe distale (215) du cathéter à la configuration de travail souhaitée lorsque le dispositif d'imagerie (400) a été enlevé du cathéter (110) et la sonde médicale est déployée dans la lumière principale (312),
**caractérisé en ce que**
dans le système de commande (14) est mémorisée une pluralité de modes (141, 142, 143, 144) pour le maintien ou le renvoi du cathéter (110) à la configuration de travail souhaitée,
le système de commande comprenant en outre un module de sélection (146) configuré pour sélectionner et activer l'un de la pluralité de modes (141, 142, 143, 144) pour le maintien ou le renvoi du cathéter en fonction d'une sélection manuelle, d'un type de sonde mécanique, ou d'un type d'activité médicale.

2. Système selon la revendication 1, dans lequel une section distale du cathéter a une largeur inférieure à environ 3 mm.

3. Système selon la revendication 2, dans lequel la lumière principale (312) dans le cathéter a une largeur qui est de plus de la moitié de la largeur de la section distale.

4. Système selon la revendication 1, dans lequel la pluralité de modes comprend un mode de position dans lequel le système de commande actionne le système mécanique pour maintenir ou renvoyer la pointe distale (215) à une position cible.

5. Système selon la revendication 1, dans lequel la pluralité de modes comprend un mode d'orientation dans lequel le système de commande actionne le système mécanique pour maintenir ou renvoyer la pointe distale (215) dans une orientation cible.

6. Système selon la revendication 1, dans lequel la pluralité de modes comprend un mode cible dans lequel le système de commande actionne le système mécanique pour contrôler une combinaison d'une position et d'une orientation de la pointe distale (215) afin de pointer la pointe distale (215) sur une position cible.

7. Système selon la revendication 1, dans lequel la pluralité de modes comprend un mode axial cible dans lequel le système de commande actionne le système mécanique pour contrôler une combinaison d'une position et d'une orientation de la pointe distale (215) de telle sorte que la pointe distale (215) reste sur ou retourne à une ligne cible.

8. Système selon la revendication 1, comprenant en outre :
une interface configurée pour déterminer si le dispositif d'imagerie (400) ou la sonde médicale est déployé(e) dans le cathéter.

9. Système selon la revendication 8, dans lequel l'interface est un périphérique de saisie par l'utilisateur qui permet à un opérateur de sélectionner un mode de fonctionnement souhaité.

10. Système selon la revendication 8, dans lequel l'interface est configurée pour détecter un type de sonde médicale déployé, et le module de sélection active l'un de la pluralité de modes en fonction du type de sonde médicale détecté.

11. Système selon la revendication 1, comprenant en outre le dispositif d'imagerie (400) qui peut être retiré du cathéter, dans lequel le dispositif d'imagerie (400) remplit la lumière principale (312) lorsque le dispositif d'imagerie (400) est déployé dans le cathéter.
